# EUROPEAN PATENT SPECIFICATION

(11) **EP 3 523 464 B1**
(45) Date of publication and mention of the grant of the patent: **24.04.2024**
(21) Application number: 17860884.0
(22) Date of filing: 05.10.2017
(51) Int. Cl.: C25D 11/00, C23C 28/00

(54) **ELECTRO-DEPOSITED CONDUCTING POLYMERS FOR THE REALIZATION OF SOLID-STATE REFERENCE ELECTRODES FOR USE IN INTRACUTANEOUS AND SUBCUTANEOUS ANALYTE-SELECTIVE SENSORS**
ELEKTROLYTISCH ABGESCHIEDENE LEITFÄHIGE POLYMERE ZUR HERSTELLUNG VON FESTKÖRPERREFERENZELEKTRODEN ZUR VERWENDUNG IN INTRAKUTANEN UND SUBKUTANEN ANALYTSELEKTIVEN SENSOREN
POLYMÈRES CONDUCTEURS ÉLECTRO-DÉPOSÉS POUR LA RÉALISATION D'ÉLECTRODES DE RÉFÉRENCE À SEMI-CONDUCTEURS DESTINÉES À ÊTRE UTILISÉES DANS DES CAPTEURS SÉLECTIFS D'ANALYTES DE TYPE INTRACUTANÉS ET SOUS-CUTANÉS

(30) Priority: 10.10.2016 US 201662406389 P
(43) Date of publication of application: 14.08.2019
(73) Proprietor: Biolinq Incorporated, San Diego, CA 92121 (US)
(72) Inventor: SATTAYASAMITSATHIT, Sirilak, San Diego, CA 92121 (US); WINDMILLER, Joshua, San Diego, CA 92121 (US); TANGNEY, Jared, San Diego, CA 92121 (US)
(74) Representative: Cooley (UK) LLP
(86) International application number: PCT/US2017/055314
(87) International publication number: WO 2018/071265

(56) References cited:
- WO-A1-2006/093422
- WO-A1-2006/093422
- CN-A- 101 915 794
- KR-A- 20160 108 111
- US-A1- 2010 200 538
- US-B2- 8 005 526
- US-B2- 8 005 526
- MANGOLD et al.: "Reference electrodes based on conducting polymer bilayers", Synthetic Metals, vol. 119 15 March 2001 (2001-03-15), pages 345-346, XP085619396, Retrieved from the Internet: URL:DOI: 10.1016/S0379-6779(00)00928-0
- CHOU et al.: "Fabrication and Investigation of Arrayed Glucose Biosensor Based on Microfluidic Framework", IEEE Sensors Journal, vol. 13, no. 11 20 May 2013 (2013-05-20), pages 4180-4187, XP011527939, Retrieved from the Internet: URL:DOI: 10.1109/JSEN.2013.2264284
- WALTMAN et al.: "Electrically conducting polymers: a review of the electropolymerization reaction, of the effects of chemical structure on polymer film properties, and of applications towards technology", Canadian Journal nf Chemistry, vol. 64, no. 1, 31 January 1986 (1986-01-31), pages 76-95, XP055214362,

## Description

### Technical Field

The present invention generally relates to sensors.

### Background Art

Prior art solutions have been concerned with the creation of quasi- or pseudo-reference electrodes - essentially metallic wires lacking a junction or internal filling solution of any sort; in certain circumstances, these metallic wires are coated with a layer of metal salt, such as a silver wire functionalized on the surface with a layer of silver chloride. Such electrodes fail to maintain a constant potential, albeit vary predictably with known conditions. Under such circumstances, the potential can be calculated and the electrode can be employed as a reference electrode. Such quasi- or pseudo-reference electrodes operate over a limited range of conditions, such as pH, ionic strength, or temperature; exceeding these boundaries and electrode behavior becomes less predictable. With regards to micro-fabricated chip-scale sensors often employed for microfluidic applications, lithographically-defined metal electrodes are utilized as a proxy for the said wires, albeit such devices suffer from the same shortcomings.

Yong Suk Choi et al., US Patent Number 6793789 for a *Reference electrode with a polymeric reference electrode membrane* discloses a polymeric reference electrode membrane comprising (a) one selected from a porous polymer or a hydrophilic plasticizer; (b) a lipophilic polymer; and optionally an adhesion-enhancing material. A reference electrode equipped with the polymeric reference electrode membrane can be shortened the preconditioning time, and extended lifetime for storage and use owing to excellent adhesion, and showed reproducibility and good yield. So, a miniaturized multi-potentiometric sensor can be fabricated comprising a solid-state reference electrode of the present invention and a set of ion-selective electrodes, thus being useful in the potentiometric fields, including clinical, environmental, food and industrial analysis.

Kinlen et al., US Patent Number 4908117 for a *Solid state reference electrode* discloses a solid state reference electrode comprising a metal/metal salt electrode, an immobilized electrolyte in contact with the metal salt, and a perfluorocarbon copolymer coating on the immobilized electrolyte to prevent migration of the electrolyte away from the electrode.

Zick et al., US Patent Number 4450842 for a *Solid state reference electrode* discloses in the reference electrode construction disclosed herein, silver and silver chloride particles are incorporated in a mixture including a fusible glass frit. After being printed on a suitable substrate, the mixture is fired to bond the silver/silver chloride composite into a conductive film on the surface of the substrate. US8005526 B2 and WO 2006/093422 A1 disclose electrodes with coated conductive polymers.

The prior art fails to disclose a viable solid-state reference electrode in micron-scale geometries.

### Summary of the Invention

The reference electrode serves as a requisite constituent of an electrochemical cell and is necessary to impart stable and controlled electrochemical reactions. Although such electrodes are commonplace in benchtop experiments, their architecture precludes these devices from integration in miniaturized platforms. Often, when attempts are made to integrate reference electrodes in micron-scale geometries, such as within semiconductor chips, the stability of the electrode is compromised. Indeed, the creation of a stable solid-state reference electrode has posed a formidable challenge to those aiming to create intracutaneously- and subcutaneously-implanted electrochemical sensors for the quantification of circulating analytes in physiological fluids.

The technology described herein relates to implantable, analyte-selective electrochemical sensors and methods for producing the same. The invention is defined in the appended claims.

One aspect of the present invention is a method according to claim 1.

Another aspect of the present invention is an analyte-selective electrochemical cell device according to claim 9.

### Brief Description of the Drawings

FIG. 1 illustrates a conventional reference electrode with the major components of the device indicated.
FIG. 2 illustrates an optical micrograph delineating two bare platinum electrodes (top row) and two conducting polymer-coated platinum electrodes (bottom row) on a silicon die.
FIG. 3 illustrates a block / process flow diagram illustrating the major constituents of the solid-state reference electrode manufacturing process.
FIG. 4 illustrates a diagrammatic representation of the prior art delineating the major functional components required for the construction of a conventional liquid junction reference electrode.
FIG. 5 illustrates an electro-polymerization of the pyrrole monomer precursor (left) in the presence of an oxidizing electrical potential to form the conducting polymer poly(pyrrole) (right).
FIG. 6 illustrates a detailed schematic representation of the electro-polymerization process of poly(pyrrole).

### Best Modes For Carrying Out The Invention

The present invention discloses a method for the synthesis of a viable, stable, solid-state reference electrode with noteworthy analytical merits. Namely, a conducting polymer is electro-deposited onto the surface of a micron- or nano-scale electrode following immersion of said electrode into a solution comprising a precursor compound to the conducting polymer, a monomer, which is simultaneously electro-polymerized to form the conducting polymer during the said electro-deposition process. This method yields a viable, stable, solid-state reference electrode. A dopant counter anion or cation is included in the solution to provide for an abundance of available charge carriers to improve electrical conduction properties and facilitate ion exchange when the as-synthesized reference electrode is utilized in physiological fluids containing like ions.

In order to impart high-fidelity sensing over extended durations, electrochemical sensors leverage reference electrodes featuring a metal electrode / liquid / semi-permeable membrane architecture to facilitate the establishment of a stable and thoroughly-characterized electrode potential from which potentials applied or measured at other electrodes in the electrochemical cell are referred. This potential remains insensitive with regards to fluctuations in (1) the chemical composition of the solution in which the reference electrode is immersed and (2) the passage of current through a separate working and counter electrode contingent. Such reference electrodes, which ideally are non-polarizable, are constructed via immersion of a metal wire (often coated with a salt of the metal) into a capillary tube filled with a saturated ionic solution ("internal filling solution") such that ion-exchange occurs between the metal (or metal salt) and the ionic solution at a precisely-defined potential under redox equilibrium. A semi-permeable membrane, located at the distal end of the capillary tube, facilitates electrical contact between the internal filling solution and the solution in which the reference electrode is immersed. Although this approach is appropriate for benchtop evaluation, the construction of such confined liquid junctions at the micron scale, especially in semiconductor manufacturing embodiments, is not easily achieved using conventional fabrication approaches. The invention disclosed provides for a means for the realization of completely solid-state reference electrodes that obviate the need for said internal filling solutions and semi-permeable membranes employed in the prior art, thereby positioning the innovation for used in micro-fabricated electrochemical cells such as sub- and intra-cutaneous analyte-selective sensors.

The present invention represents an alternative approach facilitating the synthesis of a viable solid-state reference electrode that addresses the shortcomings of the prior art while remaining amenable to highly scalable manufacturing processes. These shortcomings include:
(1) The necessity of implementing an internal filling solution, which maintains thermodynamic redox equilibrium with a metal / metal-salt electrode immersed within, thereby yielding a stable electrical potential: Limitation: (a) An internal filling solution cannot be easily implemented under micro-fabrication embodiments using conventional high-throughput manufacturing processes used in the semiconductor or microelectronics industries. (b) Mitigation: Utilization of a conducting polymer containing an embedded dopant ion to result in the establishment of a stable electrode potential that maintains thermodynamic redox equilibrium with the underlying metal electrode.
(2) The mechanical / hand assembly of conventional reference electrodes, which are multi-component in nature: (a) Limitation: Mechanical or hand assembly are not amenable to micro- or nano-scale electrode dimensions that are typically encountered in micro-fabricated systems. (b) Mitigation: Utilization of an electro-deposition process to deposit a conducting polymer film on a metallic electrode of nearly any geometry. The electro-deposition process is amenable to automated, scalable, and highly parallelized fabrication of highly uniform functional reference electrodes that obviate the need for mechanical / hand assembly, as is often done with conventional reference electrodes.
(3) The realization of micro-fabricated metal-salt electrodes: (a) Limitation: Metal salt electrodes require chemical or electrochemical processing of an elemental metal precursor and cannot be easily synthesized, as is the case with elemental metal electrodes, using micro-manufacturing equipment (b) Mitigation: The coating of an elemental metallic or metal alloy electrode with a conducting polymer containing an embedded dopant ion exhibits identical redox behavior to conventional metal-salt-coated metal electrodes when immersed in solution.
(4) Semi-permeable membrane junctions, which forms a half-cell: (a) Limitation: Conventional reference electrodes make extensive use of glass frit semi-permeable membranes, which facilitate electrical communication between the internal filling solution within the reference electrode and the surrounding solution in which said reference electrode is immersed. The fabrication of said semi-permeable membranes employing an automated or semiconductor manufacturing process is not easily achieved owing to the inability to process the material using established micro-manufacturing techniques. (b) Mitigation: The implementation of a conducting polymer emulates the semi-permeability of a conventional glass frit membrane. Said conducting polymer enables the electrical communication between the underlying metal electrode and the solution in which the as-fabricated solid-state reference electrode is immersed.
(5) Inability to scale to micron- and sub-micron dimensions: (a) Limitation: Owing to their multi-component design and extensive use of materials that are incompatible with conventional micro-fabrication and semiconductor manufacturing methods, conventional reference electrodes present difficulty when attempting to scale below centimeter-scale dimensions. (b) Mitigation: Employing a fabrication / manufacturing technique and materials that are compatible with the existing semiconductor processing infrastructure ensures that said reference electrodes can be scaled to micron- or sub-micron dimensions and integrated alongside functional electrical circuits, microfluidic systems, and micro-electromechanical systems.

The technology disclosed herein preferably requires the immersion of at least two metallic electrodes (with one selected to serve as the reference electrode) in an aqueous or non-aqueous solution comprising a monomer precursor and, optionally, a dopant ion. A constant or time-varying electrical potential or current is applied to the two metallic electrodes for a specified amount of time or until a specified amount of charge has passed.

Alternatively, the technology disclosed herein preferably requires the immersion of at least two metallic electrodes (with one selected to serve as the counter electrode) and one liquid-junction reference electrode in an aqueous or non-aqueous solution comprising a monomer precursor and, optionally, a dopant ion. A constant or time-varying electrical potential or current is applied to the two metallic electrodes (and referenced against the said reference electrode) for a specified amount of time or until a specified amount of charge has passed.

Two scenarios can occur: (1) Through an oxidative process in which one or more electrons are removed from the monomer precursor molecule, the said monomer cross-links with other monomer units to maintain charge neutrality and forms a conducting polymer chain when used as the anode in the system, otherwise referred to as anodic electro-polymerization. This forms a p-type conducting polymer (i.e. a material in which electron holes serve as the charge carrier). Simultaneously, the conducting polymer is deposited on the electrode surface, via electrostatic interaction, in a process known as anodic electro-deposition. (2) Through a reductive process in which one or more electrons are transferred to the monomer precursor molecule, the monomer cross-links with other monomer units to maintain charge neutrality and forms a conducting polymer chain when used as the cathode in the system, otherwise referred to as cathodic electro-polymerization. This forms an n-type conducting polymer (i.e. a material in which electrons serve as the charge carrier). Simultaneously, the conducting polymer is deposited on the electrode surface, via electrostatic interaction, in a process known as cathodic electro-deposition.

In each of the above-mentioned scenarios, the presence of a monoatomic or polyatomic anion or cation in the solution (containing the monomer) encourages the "doping" process. In the case of inclusion of an anion in the solution, the uptake of said ion during the electro-polymerization process for inclusion in the conducting polymer will result in a conducting polymer that is n-type. Alternatively, in the case of inclusion of a cation in the solution, the uptake of said ion during the electro-polymerization process for inclusion in the conducting polymer results in a conducting polymer that is p-type.

Under each of the above scenarios, the as-electro-deposited conducting polymer also serves a tandem function as a semi-permeable membrane that emulates the same in a conventional reference electrode without requiring an internal filling solution, hence facilitating electrical conduction between the underlying metallic electrode and the solution in which it is immersed.

Metal electrode: A metal surface, of defined geometry and surface morphology, electrically addressed by an external circuit. The metal surface preferably comprises, but is not limited to, one or more of the following elemental metals: chromium, titanium, tin, nickel, copper, silver, gold, platinum, palladium, rhodium, and iridium. Metal alloys (combination of two or more metals or metalloids) may also be employed.

Conducting polymer: An organic polymer able to conduct an electrical current. The conducting polymer is preferably doped with a counter anion(s) or cation(s) to improve electrical conductivity and provide for ion exchange with the solution in which it is immersed (such as physiological fluid - blood, plasma, extracellular fluid, intracellular fluid, interstitial fluid, cerebrospinal fluid, for example). The conducting polymer is electro-deposited onto the surface of the metal electrode to form the reference electrode.

A method for generating the electrode begins with immersion of at least two metal electrodes in a solution comprising a monomer precursor. The at least two metal electrodes are connected to a voltage or current source, which facilitates the application of a potential or the sourcing of a current to / through the electrode pair and the formation of an electrochemical cell; one of the electrodes (selected as the anode or cathode, depending on the desired redox reaction) will be functionalized as the reference electrode.

Next, application of a fixed or time-varying electrical potential or current from a voltage source or a current source to the metallic electrode contingent serves to simultaneously electro-polymerize the monomer precursor and electro-deposit a conducting polymer, synthesized from the electro-polymerization of the monomer precursor, onto the metallic electrode surface.

The inputs of the invention include a metal electrode, a solution comprising monomer precursor, a dopant ion and a constant or time-varying electrical potential or current.

In a preferred embodiment, the metal electrode has a metal, metalloid, or metal alloy surface, of defined geometry and surface morphology, electrically addressed by an external circuit. The metal surface preferably comprises, but is not limited to, one or more of the following metals: chromium, titanium, tin, nickel, copper, silver, gold, platinum, palladium, rhodium, and iridium, or alloy of two or more elemental metals.

In a preferred embodiment, the solution comprising the monomer precursor comprises, but is not limited to, one of the following monomeric compounds: aniline, acetylene, phenylene, phenylene-diamine, phenylene-vinylene, pyrrole, thiophene, and 3,4-ethylenedioxythiophene.

Ina preferred embodiment, the dopant ion (optional, in solution along with monomer) is employed as a charge carrier to provide improved electrical conduction within the conducting polymer matrix. The dopant ion also facilitates ion-exchange with like-ions in the sensing medium, such as physiological fluid. The dopant ion preferably comprises, but are not limited to, anions including chloride, sulfide, bicarbonate, sulfate, phosphate, and nitrate, or cations including hydrogen, lithium, sodium, potassium, calcium, and magnesium.

In a preferred embodiment, the constant or time-varying electrical potential or current is employed to instigate the simultaneous electro-polymerization of the monomer precursor and electro-deposition of the resultant conducting polymer onto the metal electrode. The constant or time-varying electrical potential or current is preferably applied using one of amperometry, voltammetry, and coulometry.

The output is a solid-state reference electrode, which comprises the metal electrode coated with the electro-polymerized / electro-deposited conducting polymer, optionally containing a dopant anion or cation, and it is used in conjunction with one or more separate electrodes to form an electrochemical cell.

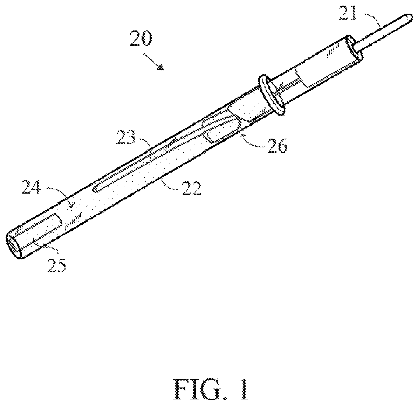

FIG. 1 illustrates a conventional reference electrode with major components of the device 20 including an electrode connector 21, a glass capillary 22, a metal wire 23, an internal ionic filling solution 24, a semi-permeable membrane 25 and a body 26. In this example, a silver/silver-chloride reference electrode, comprising a silver-chloride-coated silver wire immersed in a 1M potassium chloride solution used in conjunction with a Vycor^{®} semi-porous glass frit membrane, is shown.

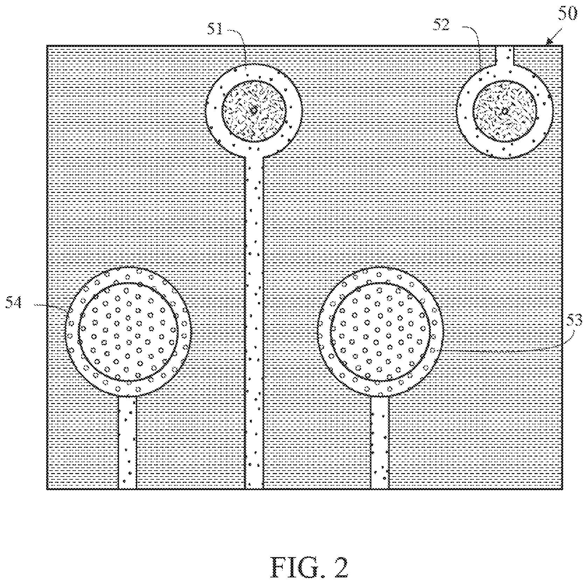

FIG. 2 illustrates an optical micrograph 50 delineating two bare platinum electrodes 51 and 52 (top row) and two conducting polymer-coated platinum electrodes 53 and 54 (bottom row) on a silicon die; both conducting polymer-coated platinum electrodes are used as solid-state, chip-scale reference electrodes. The diameter of each electrode is preferably 135 µm.

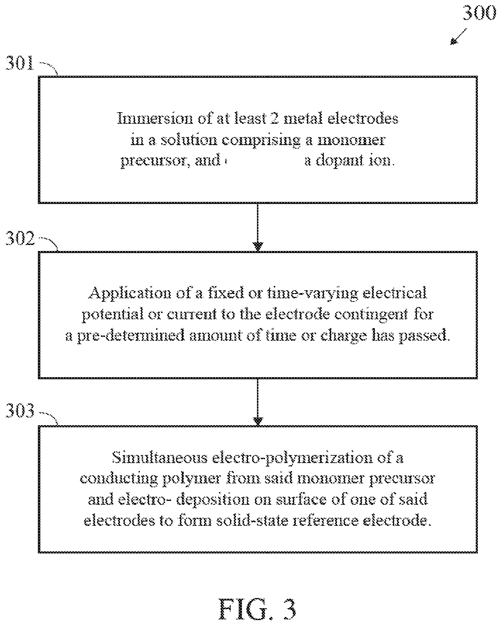

FIG. 3 illustrates a block / process flow diagram illustrating the major constituents of the solid-state reference electrode manufacturing process. A method 300 for constructing a solid-state reference electrode for use in an analyte-selective intracutaneously- or subcutaneously-implanted electrochemical cell is provided. At block 301 a metallic electrode is immersed in a solution comprising a monomer precursor. At block 302, a fixed or time-varying electrical potential or current is applied to the metallic electrode, thereby serving to simultaneously electro-polymerize the monomer precursor and electro-deposit a conducting polymer, synthesized from the electro-polymerization of said monomer precursor, onto the said metallic electrode surface. At block 303, the simultaneous electro-polymerization of a conducting polymer and the electro-deposition on the surface of at least one electrode occurs.

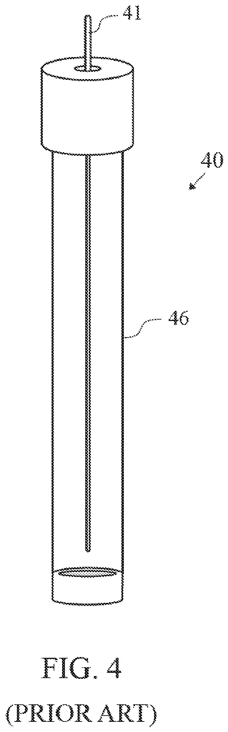

FIG. 4 illustrates a diagrammatic representation of the prior art delineating the major functional components required for the construction of a conventional liquid junction reference electrode 40 with a body 46 and an electrode connector 41.

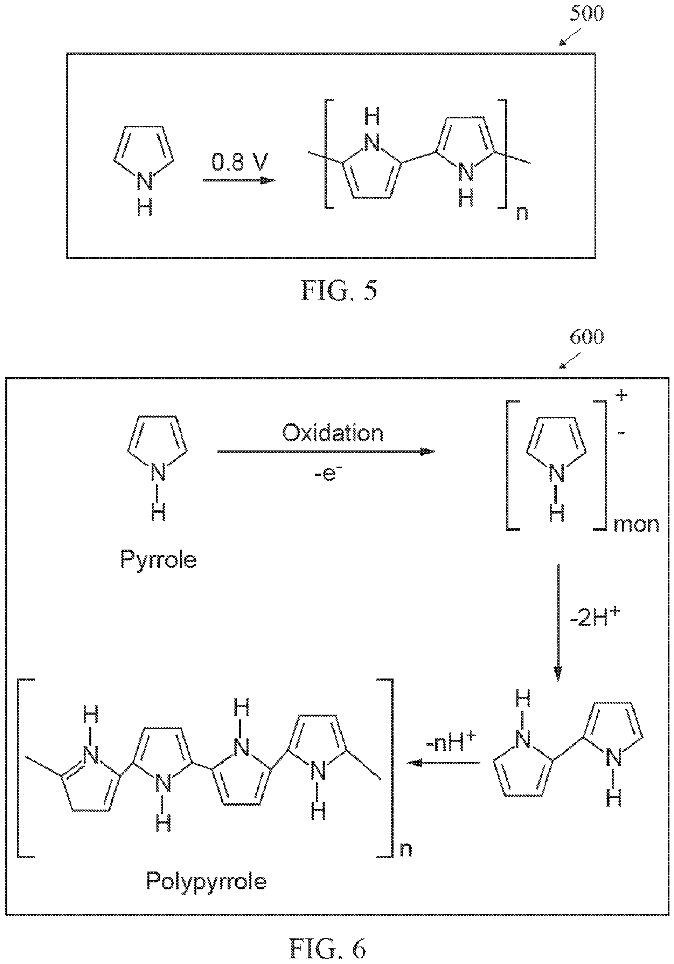

FIG. 5 illustrates an electro-polymerization process 500 of the pyrrole monomer precursor (left) in the presence of an oxidizing electrical potential to form the conducting polymer poly(pyrrole) (right).

FIG. 6 illustrates a further detailed schematic representation of the electro-polymerization process 600 of poly(pyrrole). Following the arrows: The pyrrole monomer (left, top); upon application of an oxidation potential, an electron is removed from the monomer, leading to the formation of a radical oxidized monomer carrying positive charge(right, top); with continued application of an oxidation potential of sufficient magnitude, a hydrogen bond is formed between two adjacent pyrrole monomers to maintain charge-neutrality (right, bottom); further application of said oxidation potential results in the formation of a poly(pyrrole) conducting polymer chain. The de-localized electron holes formed during the electro-polymerization process facilitate electrical conductivity (i.e. p-type charge carrier).

One embodiment is an analyte-selective intracutaneously- or subcutaneously-implanted electrochemical cell device. The device comprises a contingent of one or multiple metallic electrodes, and a layer of electro-deposited conducting polymer on said metallic electrode contingent to form a solid-state reference electrode with a stable electrode potential. The electrochemical cell device refers electrochemical measurements at a working electrode against said solid-state reference electrode.

The metallic electrode is selected from the group consisting of chromium, titanium, tin, nickel, copper, silver, gold, platinum, palladium, rhodium, and iridium.

The conducting polymer preferably comprises at least one of poly(aniline), poly(acetylene), poly(phenylene), poly(phenylene-diamine), poly(phenylene-vinylene), poly(phenylene-sulfide), poly(pyrrole), poly(thiophene), poly(3,4-ethylenedioxythiophene), or the derivatives of any of the aforementioned polymers. The conducting polymer also preferably contains a counter anion or cation to serve as the charge carrier or dopant. The monoatomic counter cation preferably includes hydrogen, lithium, sodium, potassium, calcium, or magnesium. The polyatomic counter cation preferably includes ammonium. The monoatomic counter anion preferably includes fluoride, chloride, sulfide, bromide, or iodide. The polyatomic counter anion preferably includes sulfate, bicarbonate, phosphate, nitrate, hydroxide, peroxide, acetate, carbonate, or polystyrene sulfonate. Alternatively, said conducting polymer contains an aggregate of two or more counter anions or two or more counter cations to enhance the conductivity or charge transfer properties of said conducting polymer.

Another embodiment is the incorporation of a substance encouraging proteolytic activity, anti-oxidant activity, or inhibiting microbial activity in said monomer precursor solution, along with any relevant dopant ions, in order to reduce the likelihood of biofouling, oxidative stress, or infection, respectively, when said solid-state reference electrode is implanted in a living biological system. Said proteolytic substance can comprise a protease enzyme. Said anti-oxidant substance can comprise a catalase enzyme, a superoxide dismutase enzyme, a glutathione reductase enzyme, a glutathione peroxidase enzyme, or a peroxidase enzyme. Said anti-microbial substance can comprise a lysozyme enzyme or antibiotic compound.

Another embodiment is the application of a high electrical potential or current or the application of said electrical potential or current for a sufficiently extended period of time to encourage the over-oxidation or over-reduction of said as-deposited conducting polymer. This process serves to enhance the conductivity of said conducting polymer as well as its charge-transfer properties. Said over-oxidation or over-reduction process can occur simultaneously during the electro-polymerization / electro-deposition process or following said electro-polymerization / electro-deposition process, either in the solution containing the monomer precursor (along with any dopant ions) or in a new solution entirely. Said new solution can comprise any aqueous or non-aqueous solution.

Another embodiment is the application of one or more conducting polymer layers on the surface of said conducting polymer using a process identical to that mentioned [above]. Said one or more conducting polymer layers can either contain a single counter anion, single counter cation, plurality of counter anions, plurality of counter cations, or no ions whatsoever. Said one or more conducting polymer layers can either comprise the same conducting polymer substance as the first layer or comprise a different conducting polymer substance entirely. Said one or more conducting polymer layers can either be over-oxidized, over-reduced, or be unperturbed.

Another embodiment is an electrochemical cell device comprising at least one metallic electrode, and a layer of a conducting polymer electro-deposited on the metallic electrode to form a solid-state reference electrode with a stable electrode potential. The electrochemical cell device refers electrochemical measurements at a working electrode against the solid-state reference electrode. The metallic electrode preferably possesses a two-dimensional geometric surface. The metallic electrode alternatively possesses a three-dimensional geometric surface. The metallic electrode is preferably structured to possess a shape including at least one of cylindrical, conical, circular, triangular, pyramidal, quadrilateral, or polygonal. The spatial extent of the two most distant features of the metallic electrode is preferably between 2 and 4000 micrometers.

Yet another embodiment is a method for constructing a solid-state reference electrode for use in an analyte-selective intracutaneously- or subcutaneously-implanted electrochemical cell. The method includes immersing a metallic electrode in a solution comprising a monomer precursor. The method also includes applying a fixed or time-varying electrical potential or current to the metallic electrode, thereby serving to simultaneously electro-polymerize the monomer precursor and electro-deposit a conducting polymer, synthesized from the electro-polymerization of said monomer precursor, onto the said metallic electrode surface. The monomer precursor is present in concentration preferably between 0.001 and 3.000 moles per liter (M).

The electrical potential resides in a range preferably between -1.2 and +1.2 V versus an internal or external reference electrode. The electrical current resides in a range preferably between +/-1x10(-12) and +/-1 Ampere.

The metallic electrode has a surface area between 1x(-9) and 1x(-3)m^2 [deposited conducting polymer will have same area]. The conducting polymer is preferably deposited to a thickness between 1x(-9) and 1x(-4)m on the underlying metallic electrode.

The conducting polymer is deposited in a geometry that is identical to the geometry embodied by the underlying metallic electrode.

The counter anion or counter cation is present in concentration between 1 × 10^(-4) and 3 moles per liter (M).

## Claims

1. A method for constructing a solid-state reference electrode for use in an analyte-selective intracutaneously- or subcutaneously-implanted electrochemical cell, said method comprising:
immersing a metallic electrode in a solution comprising a monomer precursor;
applying a fixed or time-varying electrical potential or current to the metallic electrode, thereby serving to simultaneously electro-polymerize the monomer precursor and electro-deposit a conducting polymer, synthesized from the electro-polymerization of said monomer precursor, onto the said metallic electrode surface;
wherein the conducting polymer comprises at least one of poly(aniline), poly(acetylene), poly(phenylene), poly(phenylenediaminie), poly(phenylene-vinylene), poly(phenylene-sulfide), poly(pyrrole), poly(thiophene) and poly(3,4-ethylenedioxythiophene);
wherein the solution contains a counter anion or cation to serve as an embedded dopant within the conducting polymer, wherein the counter anion is selected from the group consisting of chloride, sulfate, phosphate, nitrate and polystyrene sulfonate and the cation is selected from the group consisting of hydrogen, lithium, sodium, potassium, calcium and magnesium,
wherein the metallic electrode is configured as a solid-state reference electrode having a stable electrode potential when immersed in a physiological fluid.

2. The method of claim 1 wherein the metallic electrode is a micron-scale electrode having a two- or three-dimensional geometric surface and includes one or more of chromium, titanium, tin, nickel, copper, silver, gold, platinum, palladium, rhodium, and iridium.

3. The method of claim 1 wherein said monomer precursor includes at least one of aniline, acetylene, phenylene, phenylenediamine, phenylene-vinylene, pyrrole, thiophene, and 3,4-ethylenedioxythiophene.

4. The method of claim 1 further comprising forming one or more conducting polymer layers on the conducting polymer.

5. The method of claim 1 wherein said counter anion includes chloride, sulfate, phosphate, nitrate and poly styrene sulfonate.

6. The method of claim 1 wherein said fixed or time-varying electrical potential or current is applied using one of amperometry, voltammetry, and coulometry.

7. The method of claim 1 wherein said fixed or time-varying electrical potential or current is applied to encourage the formation of a radical anion or cation, thereby yielding charge carriers within the electro-deposited conducting polymer.

8. The method of claim 1 wherein said fixed or time-varying electrical potential or current is applied to reduce or oxidize the monomer precursor, thereby giving rise to charge carriers within the electro-deposited conducting polymer.

9. An analyte-selective electrochemical cell device, said device comprising:
a contingent of one or multiple metallic electrodes; and
a layer of a conducting polymer on said metallic electrode contingent to form a solid-state reference electrode with a stable electrode potential when immersed in a physiological fluid;
wherein said electrochemical cell device refers electrochemical measurements at a working electrode against said solid-state reference electrode;
wherein the conducting polymer comprises at least one of poly(aniline), poly(acetylene), poly(phenylene), poly(phenylenediaminie), poly(phenylene-vinylene), poly(phenylene-sulfide), poly(pyyrole) poly(thiophene) and poly(3,4-ethylenedioxythiophene);
wherein the conducting polymer contains a counter anion or cation to serve as an embedded dopant within the conducting polymer, wherein the counter anion is selected from the group consisting of chloride, sulfate, phosphate, nitrate, and polystyrene sulfonate and the cation is selected from the group consisting of hydrogen, lithium, sodium, potassium, calcium, and magnesium.

10. A method as claimed in claim 1:
wherein the step of applying a fixed or time-varying electrical potential or current comprises applying a time-varying electrical potential to the metallic electrode, to simultaneously electro-polymerize the monomer precursor and electro-deposit the conducting polymer onto the surface of the metallic electrode to form a solid state reference electrode;
wherein the monomer precursor comprises at least one of aniline, acetylene, phenylene, phenylenediamine, phenylene-vinylene, pyyrole, thiophene, and 3,4-ethylenedioxythiophene.

11. The device according to claim 9 wherein the metallic electrode comprises one or more of chromium, titanium, tin, nickel, copper, silver, gold, platinum, palladium, rhodium, and iridium.

12. The device according to claim 9 further comprising one or more conducting polymer layers on the conducting polymer.

13. The device according to claim 9 wherein the electro-deposited conducting polymer comprises a radical anion or cation as a charge carrier.

14. The method of any one of claims 1-8, or the device of any one of claims 9-13, wherein the electro-deposited conducting polymer is over-reduced or over-oxidated.

15. The method according to claim 4 or the device according to claim 12, wherein the one or more conducting polymer layers is a different conducting polymer substance from the conducting polymer.

## Patentansprüche

1. Verfahren zur Herstellung einer Festkörper-Referenzelektrode zur Verwendung in einer analytselektiven intrakutan oder subkutan implantierten elektrochemischen Zelle, wobei das Verfahren umfasst:
Eintauchen einer metallischen Elektrode in eine Lösung, die einen Monomervorläufer umfasst;
Anlegen eines gleichbleibenden oder zeitveränderlichen elektrischen Potentials oder Stroms an die metallische Elektrode, um dazu zu dienen, gleichzeitig den Monomervorläufer zu elektropolymerisieren und ein durch die Elektropolymerisation des Polymervorläufers synthetisiertes leitfähiges Polymer auf die Oberfläche der metallischen Elektrode elektroabzuscheiden;
wobei das leitfähige Polymer wenigstens eines von Poly(anilin), Poly(acetylen), Poly(phenylen), Poly(phenylendiamin), Poly(phenylenvinylen), Poly(phenylensulfid), Poly(pyrrol), Poly(thiophen) und Poly(3,4-ethylendioxythiophen) umfasst;
wobei die Lösung ein Gegenanion oder -kation enthält, um als ein eingebetteter Dotierstoff in dem leitfähigen Polymer zu dienen, wobei das Gegenanion ausgewählt ist aus der Gruppe bestehend aus Chlorid, Sulfat, Phosphat, Nitrat und Polystyrolsulfonat und das Kation ausgewählt ist aus der Gruppe bestehend aus Wasserstoff, Lithium, Natrium, Kalium, Calcium und Magnesium,
wobei die metallische Elektrode als eine Festkörper-Referenzelektrode mit einem stabilen Elektrodenpotential, wenn in ein physiologisches Fluid eingetaucht, gestaltet ist.

2. Verfahren gemäß Anspruch 1, wobei die metallische Elektrode eine mikrometerskalige Elektrode mit einer zwei- oder dreidimensionalen geometrischen Oberfläche ist und eines oder mehrere von Chrom, Titan, Zinn, Nickel, Kupfer, Silber, Gold, Platin, Palladium, Rhodium und Iridium enthält.

3. Verfahren gemäß Anspruch 1, wobei der Monomervorläufer wenigstens eines von Anilin, Acetylen, Phenylen, Phenylendiamin, Phenylenvinylen, Pyrrol, Thiophen und 3,4-Ethylendioxythiophen enthält.

4. Verfahren gemäß Anspruch 1, ferner umfassend Bilden einer oder mehrerer leitfähiger Polymerschichten auf dem leitfähigen Polymer.

5. Verfahren gemäß Anspruch 1, wobei das Gegenanion Chlorid, Sulfat, Phosphat, Nitrat und Polystyrolsulfonat enthält.

6. Verfahren gemäß Anspruch 1, wobei das/der gleichbleibende oder zeitveränderliche elektrische Potential oder Strom unter Verwendung von Amperometrie, Voltammetrie und Coulometrie angelegt wird.

7. Verfahren gemäß Anspruch 1, wobei das/der gleichbleibende oder zeitveränderliche elektrische Potential oder Strom angelegt wird, um die Entstehung eines radikalischen Anions oder Kations zu fördern, um Ladungsträger innerhalb des elektroabgeschiedenen leitfähigen Polymers zu ergeben.

8. Verfahren gemäß Anspruch 1, wobei das/der gleichbleibende oder zeitveränderliche elektrische Potential oder Strom angelegt wird, um den Monomervorläufer zu reduzieren oder oxidieren, um Ladungsträger innerhalb des elektroabgeschiedenen leitfähigen Polymers zu bilden.

9. Analytselektive elektrochemische Zellenvorrichtung, wobei die Vorrichtung umfasst:
eine Gruppe von einer oder mehreren metallischen Elektroden; und
eine Schicht aus einem leitfähigen Polymer auf der Gruppe von metallischen Elektroden, um eine Festkörper-Referenzelektrode mit einem stabilen Elektrodenpotential, wenn in ein physiologisches Fluid eingetaucht, zu bilden;
wobei die elektrochemische Zellenvorrichtung elektrochemische Messungen an einer Arbeitselektrode auf die Festkörper-Referenzelektrode bezieht;
wobei das leitfähige Polymer wenigstens eines von Poly(anilin), Poly(acetylen), Poly(phenylen), Poly(phenylendiamin), Poly(phenylenvinylen), Poly(phenylensulfid), Poly(pyrrol), Poly(thiophen) und Poly(3,4-ethylendioxythiophen) umfasst;
wobei das leitfähige Polymer ein Gegenanion oder -kation enthält, um als eingebetteter Dotierstoff in dem leitfähigen Polymer zu dienen, wobei das Gegenanion ausgewählt ist aus der Gruppe bestehend aus Chlorid, Sulfat, Phosphat, Nitrat und Polystyrolsulfonat und das Kation ausgewählt ist aus der Gruppe bestehend aus Wasserstoff, Lithium, Natrium, Kalium, Calcium und Magnesium.

10. Verfahren gemäß Anspruch 1:
wobei der Schritt des Anlegens eines gleichbleibenden oder zeitveränderlichen elektrischen Potentials oder Stroms Anlegen eines zeitveränderlichen elektrischen Potentials an die metallische Elektrode umfasst, um gleichzeitig den Monomervorläufer zu elektropolymerisieren und das leitfähiges Polymer auf die Oberfläche der metallischen Elektrode elektroabzuscheiden, um eine Festkörper-Referenzelektrode zu bilden;
wobei der Monomervorläufer wenigstens eines von Anilin, Acetylen, Phenylen, Phenylendiamin, Phenylenvinylen, Pyrrol, Thiophen und 3,4-Ethylendioxythiophen umfasst.

11. Vorrichtung gemäß Anspruch 9, wobei die metallische Elektrode eines oder mehrere von Chrom, Titan, Zinn, Nickel, Kupfer, Silber, Gold, Platin, Palladium, Rhodium und Iridium umfasst.

12. Vorrichtung gemäß Anspruch 9, ferner umfassend eine oder mehrere leitfähige Polymerschichten auf dem leitfähigen Polymer.

13. Vorrichtung gemäß Anspruch 9, wobei das elektroabgeschiedene leitfähige Polymer ein radikalisches Anion oder Kation als einen Ladungsträger umfasst.

14. Verfahren gemäß einem der Ansprüche 1-8 oder Vorrichtung gemäß einem der Ansprüche 9-13, wobei das elektroabgeschiedene leitfähige Polymer überreduziert oder überoxidiert ist.

15. Verfahren gemäß Anspruch 4 oder Vorrichtung gemäß Anspruch 12, wobei die eine oder mehreren leitfähigen Polymerschichten ein von dem leitfähigen Polymer verschiedener Polymerstoff ist.

## Revendications

1. Procédé de construction d'une électrode de référence à l'état solide pour une utilisation dans une cellule électrochimique sélective pour un analyte implantée par voie intra-cutanée ou sous-cutanée, ledit procédé comprenant :
l'immersion d'une électrode métallique dans une solution comprenant un précurseur monomérique ;
l'application d'un potentiel ou d'un courant électrique fixe ou variant dans le temps à l'électrode métallique, servant ainsi à électropolymériser simultanément le précurseur monomérique et à électrodéposer un polymère conducteur, synthétisé à partir de l'électropolymérisation dudit précurseur monomérique, sur ladite surface de l'électrode métallique ;
dans lequel le polymère conducteur comprend au moins l'un parmi poly(aniline), poly(acétylène), poly(phénylène), poly(phénylènediamine), poly(phénylène-vinylène), poly(sulfure de phénylène), poly(pyrrole), poly(thiophène) et poly(3,4-éthylènedioxythiophène) ;
dans lequel la solution contient un contre-anion ou un cation pour servir de dopant incorporé dans le polymère conducteur, dans lequel le contre-anion est sélectionné dans le groupe constitué par chlorure, sulfate, phosphate, nitrate et poly(sulfonate de styrène) et le cation est sélectionné dans le groupe constitué par hydrogène, lithium, sodium, potassium, calcium et magnésium,
dans lequel l'électrode métallique est configurée comme une électrode de référence à l'état solide ayant un potentiel d'électrode stable lorsqu'elle est immergée dans un liquide physiologique.

2. Procédé selon la revendication 1, dans lequel l'électrode métallique est une électrode à l'échelle du micron ayant une surface géométrique bidimensionnelle ou tridimensionnelle et comporte l'un ou plusieurs parmi le chrome, le titane, l'étain, le nickel, le cuivre, l'argent, l'or, le platine, le palladium, le rhodium et l'iridium.

3. Procédé selon la revendication 1, dans lequel ledit précurseur monomérique comporte au moins l'un parmi l'aniline, l'acétylène, le phénylène, la phénylènediamine, le phénylène-vinylène, le pyrrole, le thiophène et le 3,4-éthylènedioxythiophène.

4. Procédé selon la revendication 1 comprenant en outre la formation d'une ou plusieurs couches de polymère conducteur sur le polymère conducteur.

5. Procédé selon la revendication 1, dans lequel ledit contre-anion comporte chlorure, sulfate, phosphate, nitrate et poly(sulfonate de styrène).

6. Procédé selon la revendication 1, dans lequel ledit potentiel ou courant électrique fixe ou variant dans le temps est appliqué en utilisant l'une parmi l'ampérométrie, la voltampérométrie et la coulométrie.

7. Procédé selon la revendication 1, dans lequel ledit potentiel ou courant électrique fixe ou variant dans le temps est appliqué pour encourager la formation d'un anion ou d'un cation radicalaire, produisant ainsi des porteurs de charges dans le polymère conducteur électrodéposé.

8. Procédé selon la revendication 1, dans lequel ledit potentiel ou courant électrique fixe ou variant dans le temps est appliqué pour réduire ou oxyder le précurseur monomérique, donnant ainsi lieu à des porteurs de charges dans le polymère conducteur électrodéposé.

9. Dispositif à cellule électrochimique sélective pour un analyte, ledit dispositif comprenant :
un contingent d'une ou plusieurs électrodes métalliques ; et
une couche de polymère conducteur sur ledit contingent d'électrodes métalliques pour former une électrode de référence à l'état solide avec un potentiel d'électrode stable lorsqu'elle est immergée dans un liquide physiologique ;
dans lequel ledit dispositif à cellule électrochimique réfère des mesures électrochimiques à une électrode de travail contre ladite électrode de référence à l'état solide ;
dans lequel le polymère conducteur comprend au moins l'un parmi poly(aniline), poly(acétylène), poly(phénylène), poly(phénylènediamine), poly(phénylène-vinylène), poly(sulfure de phénylène), poly(pyrrole) poly(thiophène) et poly(3,4-éthylènedioxythiophène) ;
dans lequel le polymère conducteur contient un contre-anion ou un cation servant de dopant incorporé dans le polymère conducteur, dans lequel le contre-anion est sélectionné dans le groupe constitué par chlorure, sulfate, phosphate, nitrate, et poly(sulfonate de styrène) et le cation est choisi dans le groupe constitué par hydrogène, lithium, sodium, potassium, calcium et magnésium.

10. Procédé selon la revendication 1 :
dans lequel l'étape d'application d'un potentiel ou d'un courant électrique fixe ou variant dans le temps comprend l'application d'un potentiel électrique variant dans le temps à l'électrode métallique, afin d'électropolymériser simultanément le précurseur monomérique et d'électrodéposer le polymère conducteur sur la surface de l'électrode métallique pour former une électrode de référence à l'état solide ;
dans lequel le précurseur monomérique comprend au moins l'un parmi l'aniline, l'acétylène, le phénylène, la phénylènediamine, le phénylène-vinylène, le pyrrole, le thiophène et le 3,4-éthylènedioxythiophène.

11. Dispositif selon la revendication 9, dans lequel l'électrode métallique comprend l'un ou plusieurs parmi le chrome, le titane, l'étain, le nickel, le cuivre, l'argent, l'or, le platine, le palladium, le rhodium et l'iridium.

12. Dispositif selon la revendication 9 comprenant en outre une ou plusieurs couches de polymère conducteur sur le polymère conducteur.

13. Dispositif selon la revendication 9, dans lequel le polymère conducteur électrodéposé comprend un anion ou un cation radicalaire comme porteur de charge.

14. Procédé selon l'une quelconque des revendications 1-8, ou dispositif selon l'une quelconque des revendications 9-13, dans lequel le polymère conducteur électrodéposé est sur-réduit ou sur-oxydé.

15. Procédé selon la revendication 4 ou dispositif selon la revendication 12, dans lequel la ou les couches de polymère conducteur est une substance de polymère conducteur différente du polymère conducteur.
